# EUROPÄISCHE PATENTSCHRIFT

(11) **EP 3 278 770 B1**
(45) Veröffentlichungstag und Bekanntmachung des Hinweises auf die Patenterteilung: **03.04.2019**
(21) Anmeldenummer: 17189382.9
(22) Anmeldetag: 03.07.2013
(51) Int. Cl.: A61F 2/60, A61F 2/68, A61F 2/70, A61F 2/50, A61F 2/64, A61F 2/66, A61F 2/74

(54) **VERFAHREN ZUR STEUERUNG EINER ORTHOPÄDIETECHNISCHEN GELENKEINRICHTUNG**
METHOD FOR CONTROLLING AN ORTHOPEDIC PIVOT LINK DEVICE
PROCÉDÉ DE COMMANDE D'UN DISPOSITIF D'AIDE TECHNIQUE ORTHOPÉDIQUE D'ARTICULATION

(30) Priorität: 03.07.2012 DE 102012013140
(43) Veröffentlichungstag der Anmeldung: 07.02.2018
(62) Teilanmeldung aus: 13737135.7
(73) Patentinhaber: Ottobock SE & Co. KGaA, 37115 Duderstadt (DE)
(72) Erfinder: Pusch, Martin, 37115 Duderstadt (DE); Will, Christian, 37077 Göttingen (DE)
(74) Vertreter: Gramm, Lins & Partner Patent- und Rechtsanwälte PartGmbB

(56) Entgegenhaltungen:
- EP-A2- 1 417 942
- WO-A1-2010/064063
- WO-A2-2007/103579
- DE-A1-102008 045 113
- US-A1- 2006 249 315

## Beschreibung

Die Erfindung betrifft ein Verfahren zur Steuerung einer orthopädietechnischen Gelenkeinrichtung einer unteren Extremität sowie eine orthopädietechnische Gelenkeinrichtung als solche, die Gelenkeinrichtung weist ein Oberteil und ein gelenkig daran gelagertes Unterteil auf, zwischen dem Oberteil und dem Unterteil ist eine Dämpfereinrichtung angeordnet, über die die Extensionsdämpfung und/oder die Flexionsdämpfung der Schwenkbewegung bewirkt wird, wobei während des Gehens kinetische Energie aus der Relativbewegung zwischen dem Unterteil und dem Oberteil umgewandelt und gespeichert und dem Gelenk wieder zugeführt wird, um die Relativbewegung zu unterstützen.

Orthopädietechnischen Gelenkeinrichtungen einer unteren Extremität sind beispielsweise Orthesen oder Prothesen. Insbesondere bei Prothesen, die ein natürliches Kniegelenk ersetzen, ist es vorteilhaft und vorgesehen, dass eine aktive Beeinflussung des Flexions- und Extensionswiderstandes im Verlaufe des Bewegungszyklus erfolgt, um das Verhalten der Gelenkeinrichtung an das Bewegungsverhalten oder an andere Einflüsse anzupassen.

Darüber hinaus ergibt es motorisch angetriebene Prothesen oder Orthesen, deren Antriebsmotoren dazu dienen eine Flexion oder Extension der jeweiligen Gelenkeinrichtung auszuführen.

Die WO 2007/025116 A2 beschreibt eine Protheseneinrichtung mit einem elektronisch gesteuerten Prothesenknie mit einer regenerativen Bremseinrichtung. In bestimmten Situationen wird die beim Gehen vorhandene kinetische Energie in elektrische Energie umgewandelt und gespeichert. In anderen Situationen ist vorgesehen, dass das Gangverhalten unterstützt oder vollständig gesteuert wird. Ein elektronisches Steuerungssystem ist vorgesehen, um den Betrieb der Protheseneinrichtung zu steuern und erzeugte elektrische Energie zu verteilen. Überschüssige elektrische Energie kann einem Akkumulator oder Kondensator gespeichert werden und zu einem geeigneten Zeitpunkt zur Bewegungsunterstützung abgerufen werden. Die Energiespeichereinrichtungen sind bei den Prothesen oder Orthesen groß und schwer, um ausreichend Kapazität zu haben, damit eine effektive Bewegungsunterstützung erfolgen kann. Darüber hinaus besteht die Möglichkeit, dass durch die aktive Verlagerung des Oberteils zu dem Unterteil mit entsprechend starken Antrieben der Nutzer in für ihn nicht mehr kontrollierbare Situationen gebracht wird.

Die EP 439 028 B1 beschreibt eine Schwenkverbindung zwischen zwei Teilen eines orthopädietechnischen Hilfsmittels in Gestalt eines polyzentrischen Prothesenkniegelenkes, bei der unter Einwirkung einer äußeren Kraft ein Gelenkglied längenveränderlich ausgebildet ist. Die Längenveränderung des Gelenkgliedes kann federelastisch ausgebildet sein, so dass unmittelbar nach Verringerung oder Wegfall der äußeren Kraft das Gelenkglied seine ursprünglich Länge wieder einnimmt.

Ein Verfahren zur Steuerung einer orthopädietechnischen Gelenkeinrichtung gemäß des Oberbegriffs von Anspruch 1 ist aus der DE 10 2008 045113 A1 bekannt.

Aufgabe der vorliegenden Erfindung ist es daher, ein Verfahren und eine Einrichtung bereitzustellen, mit denen es möglich ist, eine Verbesserung des Gangbildes zu erreichen, ohne dass der Nutzer gefährdet wird und er eine schwere Gelenkeinrichtung tragen muss.

Erfindungsgemäß wird diese Aufgabe durch ein Verfahren mit den Merkmalen des Hauptanspruches und eine orthopädietechnische Gelenkeinrichtung mit den Merkmalen des nebengeordneten Anspruchs gelöst. Vorteilhafte Ausgestaltungen und Weiterbildungen der Erfindung sind in den jeweils abhängigen Unteransprüchen, der Beschreibung und in den Figuren aufgeführt.

Das erfindungsgemäße Verfahren zur Steuerung einer orthopädietechnischen Einrichtung einer unteren Extremität, bei der die Gelenkeinrichtung ein Oberteil und ein gelenkig daran gelagertes Unterteil aufweist und zwischen dem Oberteil und dem Unterteil eine Energieumwandlungseinrichtung angeordnet ist, über die während des Gehens kinetische Energie aus der Relativbewegung zwischen dem Unterteil und dem Oberteil umgewandelt und/oder gespeichert und dem Gelenk wieder zugeführt wird, um die Relativbewegung zu unterstützen, sieht vor, dass innerhalb eines Bewegungszyklus kinetische Energie umgewandelt und/oder gespeichert wird und innerhalb desselben Bewegungszyklus der Gelenkeinrichtung zeitversetzt als kinetische Energie wieder zugeführt wird. In einem typischen Bewegungszyklus einer orthopädietechnischen Gelenkeinrichtung einer unteren Extremität, also in einem typischen Schrittzyklus, existieren Phasen, in denen überschüssige Energie umgewandelt werden muss, aber auch Phasen, in denen eine Unterstützung mit kinetischer Energie sinnvoll ist. Es ist daher vorgesehen, dass überschüssige Energie, die zum Abbremsen einer Komponente der Gelenkeinrichtung gespeichert oder umgewandelt und gespeichert wird, im selben Schrittzyklus an geeigneter Stelle wieder zugeführt wird, wobei zwischen dem Speichern bzw. Umwandeln und Speichern und der erneuten Zuführung als kinetische Energie ein Zeitversatz vorliegt, also sich die Rückführung nicht unmittelbar an die Speicherung anschließt. Aufgrund der Zuführung der umgewandelten oder gespeicherten Energie in demselben Bewegungszyklus ist ein großer Akkumulator oder Kondensator unnötig, da nur relativ geringe Energiemengen vorhanden sind. Dadurch wird Gewicht eingespart und die Gelenkeinrichtung kann leicht gehalten werden. Die Einrichtung zur Umwandlung und/oder Speicherung kinetischer Energie kann als Teil einer Dämpfereinrichtung ausgebildet sein und einen Teil der Extensionsdämpfung und/oder die Flexionsdämpfung der Schwenkbewegung bewirken, wenn Energie umgewandelt oder gespeichert wird. Die Einrichtung kann als Hydraulikdämpfer und/oder Pneumatikdämpfer ausgebildet sein, ebenfalls kann eine Ausgestaltung als Generator vorgesehen sein. Möglich sind auch Kombinationen der vorbeschriebenen Einrichtungen miteinander. Die Einrichtung zur Umwandlung und/oder Speicherung kinetischer Energie kann neben herkömmlichen Dämpfereinrichtungen vorgesehen sein. Die übrigen Dämpfungskomponenten, in der Regel pneumatische oder hydraulische Dämpfer, bleiben weiter erhalten, jedoch ist zusätzlich entweder ein Kraftspeicher oder ein Generator, der umschaltbar als Motor betreibbar ist, vorgesehen, um an den geeigneten Stellen innerhalb der Bewegung kinetischer Energie aus dem System zu nehmen oder dem System zuzuführen. Aufgrund der Tatsache, dass das hydraulische oder pneumatische Dämpfungssystem einen großen Anteil der Energie dissipiert, kann das System zur Unterstützung der Bewegung klein und leicht gehalten werden. Der Zeitpunkt der Energieumwandlung und Rückführung wird über eine Steuereinrichtung festgelegt.

Eine Weiterbildung der Erfindung sieht vor, dass kinetische Energie während einer Extensionsbewegung umgewandelt und/oder gespeichert wird. Die erneute Zuführung kinetischer Energie kann bei der Einleitung der Schwungphase zur Unterstützung der Schwungphasenflexion erfolgen. Ebenfalls kann nach der Zehenablösung ("toe off") während des Schrittes zur Aufrechterhaltung der Flexionsgeschwindigkeit kinetische Energie wieder zugeführt werden. Auch ist vorgesehen, dass nach dem Erreichen des maximalen Flexionswinkels, der beispielsweise durch eine Bewegungsumkehr oder durch einen Geschwindigkeitssensor ermittelt werden kann, zur Unterstützung der Extensionsbewegung Energie zugeführt werden. Auch kann nach der Flexionsbewegung nach dem initialen Fersenkontakt, also nach der Standphasenflexion, zur Unterstützung der Extensionsbewegung die kinetische Energie erneut zugeführt werden. Grundsätzlich ist es möglich, dass die Zuführung nur bei einer einzelnen Phase der Schrittbewegung erfolgt, alternativ dazu kann zu mehreren oder allen beschriebenen Phasen oder Zeitpunkten der Schrittbewegung eine Zuführung kinetischer Energie erfolgen.

Weiterhin kann vorgesehen sein, dass kinetische Energie während der Flexionsbewegung umgewandelt und/oder gespeichert wird und zur Einleitung der Schwungphase zur Unterstützung der Flexionsbewegung und/oder zur Aufrechterhaltung der Beugegeschwindigkeit nach der Zehenablösung wieder zugeführt wird. Daher kann vorgesehen sein, dass die kinetische Energie nach Einleitung der Schwungphase umgewandelt und/oder gespeichert wird und erst nach Erreichen der maximalen Flexionsgeschwindigkeit zur Unterstützung der Beugebewegung wieder zugeführt wird. Die Erreichung der maximalen Flexionsgeschwindigkeit kann durch einen Geschwindigkeitssensor oder einen Beschleunigungssensor ermittelt werden.

In der Regel wird die Extensionsbewegung vor Erreichen der maximalen Extension abgebremst, um den Impuls zu mindern, der bei einem ungebremsten Aufprallen in den Extensionsanschlag vorliegt. Diese kinetische Energie kann umgewandelt und/oder gespeichert und zur Einleitung und/oder Unterstützung der Flexion der Gelenkeinrichtung wieder zugeführt werden. Während der Schwungphase der Gelenkeinrichtung kann kinetische Energie zur Erhöhung oder zur Aufrechterhaltung der Extensionsgeschwindigkeit zugeführt werden, um das Vorbringen des Unterteils zu erleichtern und zu unterstützen.

Weiterhin ist vorgesehen, dass kinetische Energie während der Standphase zu Beginn der Standphasenflexion bei Fersenbelastung, vor dem Erreichen des Extensionsanschlages und/oder nach der Einleitung der Standphasenflexion bei Vorfußbelastung umgewandelt und/oder gespeichert wird. Insbesondere kann die kinetische Energie bei dem initialen Fersenstoß umgewandelt und/oder gespeichert werden und zur Einleitung und/oder Unterstützung der Flexionsbewegung nach Erreichen eines Extensionsanschlages wieder zugeführt werden.

Bei zunehmender Gehgeschwindigkeit kann vorgesehen sein, dass weniger kinetische Energie dem Gelenk zugeführt wird, um das System nicht selbst zu verstärken und somit den Nutzer der orthopädietechnischen Gelenkeinrichtung dazu zu bringen, immer schneller zu gehen.

Die kinetische Energie kann in elektrische Energie umgewandelt und zwischengespeichert oder in potentielle Energie umgewandelt und zwischengespeichert werden, beispielsweise durch Aufladen eines Kraftspeichers, beispielsweise einer Feder oder eines hydraulischen oder pneumatischen Druckspeichers.

Es ist weiterhin vorgesehen, dass die umgewandelte Energie in einem Bewegungszyklus vollständig der Gelenkeinrichtung wieder zugeführt wird, so dass keine über den Bewegungszyklus hinausgehende Speicherung der umgewandelten Energie erfolgt. Dadurch wird sichergestellt, dass nur die während des einen Bewegungszyklus vorliegende und umgewandelte Energie dem System wieder als kinetische Energie zugeführt werden kann. Dies kann dadurch erfolgen, dass nach der erkannten Wiederkehr einer charakterisierenden Größe des Bewegungszyklus die bis dahin gespeicherte Energie dissipiert wird oder die Energiemenge laufend über den letzten Bewegungszyklus kontrolliert wird.

Weiterhin kann vorgesehen sein, dass die Speicherung und/oder Umwandlung der kinetischen Energie nur in vorbestimmten Phasen während eines Bewegungszyklus durchgeführt wird, also dass die Energieumwandlungseinrichtung nicht ständig arbeitet, um einen Teil der kinetischen Energie aus der Bewegung umzuwandeln, zu Speichern oder wider der Gelenkeinrichtung als kinetische Energie zuzuführen. Dazu werden entweder bestimmte Bewegungsphasen festgelegt, in denen immer eine Umwandlung, Speicherung oder Rückführung stattfindet, beispielsweise über eine mechanische oder elektrische Steuerungseinrichtung, oder die Zeitpunkte und Zeiträume, in denen eine Umwandlung, Speicherung oder Rückführung stattfindet, werden aufgrund einer Analyse der Bewegung anhand von Kenngrößen für jeden Bewegungszyklus oder für eine vorbestimmte oder errechnete Anzahl von Bewegungszyklen festgelegt. Dies kann beispielsweise über eine elektronische Steuereinrichtung erfolgen.

Eine Weiterbildung des erfindungsgemäßen Verfahrens, bei dem während einer Verschwenkung des Oberteils relativ zu dem Unterteil mechanische Arbeit aus der Relativbewegung umgewandelt und in zumindest einem Energiespeicher gespeichert und der Gelenkeinrichtung zeitversetzt wieder zugeführt wird, um die Relativbewegung zu unterstützen, sieht vor, dass die gespeicherte Energie rückgewandelt und die Zufuhr mechanischer Arbeit zur und während der Unterstützung der Relativbewegung kontrolliert erfolgt. Bei einer Freigabe von Energie aus einem Energiespeicher, beispielsweise einer Feder, wird die gespeicherte Energie gemäß dem Stand der Technik schlagartig der Gelenkeinrichtung, also dem System aus Oberteil und Unterteil und gelenkiger Lagerung, zugeführt, so dass über einen sehr kurzen Zeitraum eine hohe Energiemenge eingeleitet wird. Es ist vorgesehen, dass die gespeicherte Energie kontrolliert dem System wieder zugeführt und in mechanische Arbeit und Unterstützung der Verlagerung von Oberteil zu Unterteil umgewandelt wird, um über einen längeren Zeitraum die Bewegung zu unterstützen, so dass eine an den natürlichen Bewegungsablauf angenäherte Bewegung der prothetischen oder orthetischen Einrichtung erfolgen kann. Eine Anpassung an veränderte Gangmuster, die Geschwindigkeiten oder unterschiedliche Patienten ist gemäß dem Stand der Technik nur außerordentlich aufwendig auszuführen, indem speziell angepasste Federn eingesetzt werden, was für den täglichen Einsatz unpraktikabel ist. Erfindungsgemäß wird dagegen die Energieabgabe in das System kontrolliert, so dass über einen vergleichsweise langen Zeitraum die benötigte Energiemenge eingespeist werden kann, um das Gangbild wie gewünscht zu beeinflussen.

Die Zufuhr der mechanischen Arbeit kann dadurch verändert werden, dass dem Energiespeicher extern Energie zugeführt oder entzogen wird. Handelt es sich bei dem Energiespeicher um eine Feder, kann die Zufuhr der Energie dadurch erfolgen, dass die Feder nachgespannt wird, das Entziehen oder das Verringern der Energiemenge kann dadurch erfolgen, dass die Feder entspannt wird, beispielsweise durch Verlagerung eines Federwiderlagers. Ist der Energiespeicher als elektrischer Energiespeicher, beispielsweise Kondensator, Batterie oder Akkumulator ausgebildet, kann die Veränderung der Energiemenge durch Aktivierung eines Generators oder Einleitung aus einem zweiten Energiespeicher erfolgen, die Verringerung der Energiemenge durch Anschließen eines Verbrauchers oder Umleitung in einen zweiten Speicher für elektrische Energie erfolgen.

Eine Weiterbildung der Erfindung sieht vor, dass dem Energiespeicher ein Aktuator zugeordnet ist, über den der Energiespeicher auf ein Mindestniveau befüllt oder gebracht wird, wenn die Relativbewegung dazu nicht ausreicht. Sollte die durch die Bewegung verfügbare Energie nicht ausreichen, um den Energiespeicher für den nächsten Schritt oder den Bewegungsablauf mit ausreichend Energie zu versorgen, wobei die Mindestmenge in Abhängigkeit von der Gehgeschwindigkeit, der Gehsituation und den individuellen Gegebenheiten eines Patienten abhängen, ist erfindungsgemäß vorgesehen, dass während des Gehens und vor der Rückführung von Energie zur Unterstützung der Relativbewegung der Energiespeicher auf ein festgelegtes Niveau aufgefüllt wird, beispielsweise durch Spannen einer Feder oder durch Antreiben eines Generators, der den elektrischen Energiespeicher auflädt.

Um den Zeitpunkt der Bewegungsunterstützung präzise bestimmen zu können ist erfindungsgemäß vorgesehen, dass dem Energiespeicher eine Freigabeeinrichtung zugeordnet ist, über die die Energie aus dem Energiespeicher teilweise oder vollständig freigegeben wird. Die Freigabeeinrichtung bestimmt den Zeitpunkt der Energieabgabe, die Dauer und den Verlauf der Energieabgabe wird bei einer vollständigen Freigabe nicht über die Freigabeeinrichtung gesteuert, sondern über Veränderungen in dem Energiespeicher, also durch Entzug oder Zufuhr von Energie. Bei einer teilweisen Freigabe erfolgte eine Reduzierung der abgegebenen Energiemenge, so dass das Anfangsniveau der Bewegungsunterstützung eingestellt werden kann. Über eine teilweise Freigabe kann eine Anpassung beispielsweise an Gehgeschwindigkeiten, Patienten oder Gehsituationen vorgenommen werden, die Feinbeeinflussung der Unterstützung erfolgt über die Veränderung im Energiespeicher.

Die Energie kann als mechanische Arbeit in Abhängigkeit von zumindest einem folgenden Kriterium oder einer Kombination mehrerer folgenden Kriterien zugeführt werden, nämlich der Winkelstellung des Oberteils zu dem Unterteil, der Position des Oberteils und/oder des Unterteils im Raum, einer Winkelgeschwindigkeit des Oberteils und/oder des Unterteils, der Relativgeschwindigkeit zwischen Oberteil und Unterteil, der Belastungssituation und/oder der Beschleunigung des Oberteils und/oder des Unterteils. Dadurch ist es möglich, dass eine möglichst genaue zeitliche und mengenmäßige Unterstützung der Bewegung erfolgt. Die Stellungen der Oberteile und Unterteile zueinander und im Raum können durch Winkelsensoren oder Inertialsensoren bestimmt werden, die Geschwindigkeiten zueinander oder innerhalb des Raumes durch Beschleunigungssensoren oder einer Kombination aus Winkelsensor und Beschleunigungssensor. Über die Sensoren lässt sich nicht nur der Zeitpunkt der Freigabe der Energie, sondern auch die jeweilige Gangsituation, die Gehgeschwindigkeit sowie die aktuelle Position der jeweiligen Komponenten zueinander oder im Raum bestimmen, worüber es ermöglicht wird, die Menge und den Verlauf der Energiezufuhr zur Unterstützung der Bewegung zu bestimmen und zu steuern.

Eine Weiterbildung der Erfindung sieht vor, dass die Energie dem Energiespeicher in Abhängigkeit von zumindest einem oder mehreren der oben aufgeführten Kriterien zugeführt oder entzogen wird, um die kontrollierte Steuerung der Bewegung durchzuführen.

Der Zeitpunkt des Ineingrifftretens der Umwandlungseinrichtung zur Veränderung der umzuwandelnden Energiemenge und/oder der zugeführten Energiemenge kann verstellt werden, so dass beispielsweise in Abhängigkeit von der Gehgeschwindigkeit, der Gehsituation oder den individuellen Parametern des Patienten eingestellt werden kann, wie groß die zu speichernde Energiemenge ist oder wie groß die abzugebende Energiemenge sein muss. Bei einer gewünschten großen Energiemenge ist es vorgesehen, dass ein möglichst früher Eingriff bei der Umwandlung stattfindet, so dass beispielsweise ein Generator sehr früh und sehr lang angetrieben wird oder eine Feder sehr weit vorgespannt wird, um die mechanische Arbeit beim Gehen, beispielsweise beim Fersenauftritt in der Standphasenflexion, maximal in die Lageenergie einer Feder oder elektrische Energie eines Akkumulators oder eines Kondensators umzuwandeln. Wird der Zeitpunkt des Eingriffs bei der Rückwandlung verstellt, beispielsweise durch Verlagerung eines Anschlages oder einer winkelabhängigen Freigabe, wird die Energie zu einem späteren Zeitpunkt des Schrittes eingeleitet, wodurch sich eine Veränderung des Gangbildes erreichen lässt. Der Energiespeicher kann von einem Aktuator aufgeladen werden, wenn die Umwandlungseinrichtung nicht aufgrund der Relativbewegung zwischen dem Oberteil und dem Unterteil aktiv ist, so dass der Aktuator nicht gegen die Relativbewegung arbeiten muss. Darüber hinaus hat die zeitliche Einordnung der Aufladung des Energiespeichers durch den Aktuator in eine Phase, in der keine mechanische Arbeit aus der Gelenkeinrichtung umgewandelt wird, den Vorteil, dass über einen langen Zeitraum Energie eingespeichert werden kann, was dazu führt, dass der Aktuator entsprechend klein dimensioniert werden kann, um über einen großem Zeitraum die gewünschte Menge bereitstellen zu können. Wird beispielsweise eine Feder über einen Motor als Aktuator gespannt, kann dieser klein ausgelegt werden und mit einer Übersetzung mit der Feder gekoppelt werden, so dass über einen vergleichsweise großen Zeitraum die Feder gespannt werden kann. Gleiches gilt für die Umwandlung und Speicherung elektrischer Energie.

Eine Weiterbildung der Erfindung sieht vor, dass die Relativbewegung zusätzlich zu der Beeinflussung des Energiespeichers über eine Dämpfereinrichtung beeinflusst wird, so dass nicht ausschließlich über den Energiespeicher die Steuerung erfolgen muss, was dazu führt, dass eine große Variationsmöglichkeit bei der Beeinflussung des Gangbildes vorliegt. Darüber hinaus können Belastungsspitzen über eine zusätzliche Dämpfereinrichtung leichter aufgefangen werden.

Die orthopädietechnische Gelenkeinrichtung der unteren Extremität mit einem Oberteil und einem gelenkig daran gelagerten Unterteil und eine Einrichtung zur Umwandlung und/oder Speicherung kinetischer Energie sieht vor, dass die Speichereinrichtung eine Umwandlungs- und/oder Speicherungsrate aufweist, die umgekehrt proportional zu der Verschwenkgeschwindigkeit des Unterteils relativ zu dem Oberteil ist. Dadurch wird gewährleistet, dass trotz der eigentlich zunehmenden, umzuwandelnden kinetischen Energie keine Selbstverstärkung erfolgt, so dass das System der Gelenkeinrichtung stabil bleibt.

Es können ein Generator zur Erzeugung elektrischer Energie und ein Motor zum Antreiben des Unterteils vorgesehen sein, wobei dem Generator eine geschwindigkeitsabhängige Kupplung vorgeschaltet ist, die den Generator mit einer Antriebseinrichtung verbindet. Je höher die Bewegungsgeschwindigkeit ist, desto geringer ist die Leistung, die über die Kupplung übertragen wird, so dass die umgekehrte Proportionalität der Energieerzeugung im Verhältnis zur Verschwenkgeschwindigkeit über die Kupplung realisiert wird.

In der Gelenkeinrichtung kann eine Hydraulikdämpfereinheit zur Dämpfung der Verschwenkbewegung zwischen dem Unterteil und dem Oberteil angeordnet sein, wobei das Hydraulikfluid der Hydraulikdämpfereinheit als Aktuator zur Trennung der Kupplung dient. Die Kupplung kann als eine geschwindigkeitsabhängige Rutschkupplung ausgebildet sein.

Als Speichereinrichtung für die kinetische Energie kann ein Schwungrad oder ein Druckspeicher vorgesehen sein. Die Gelenkeinrichtung ist bevorzugt als ein Hüftgelenk, Kniegelenk oder Fußgelenk einer Prothese oder Orthese ausgebildet. Der Energiespeicher kann auch als Feder oder Akkumulator ausgebildet sein, wobei unter einem Akkumulator auch ein Kondensator oder eine aufladbare Batterie verstanden wird.

Eine Weiterbildung der orthopädietechnischen Gelenkeinrichtung sieht vor, dass dem Energiespeicher ein Aktuator zugeordnet ist, der während der Unterstützung der Relativbewegung dem Energiespeicher kontrolliert Energie zuführt oder entzieht. Durch die kontrollierte Zufuhr oder den kontrollierten Entzug von Energie aus dem Energiespeicher über einen Aktuator ist eine besonders einfache und zuverlässige Beeinflussung des Gangbildes bei semiaktiven Gelenkeinrichtungen, insbesondere bei semiaktiven Prothesenkniegelenkten möglich. Die Umwandlungseinrichtung kann als Feder oder Generator ausgebildet sein, um die mechanische Arbeit, die bei einer Relativbewegung zwischen dem Oberteil und dem Unterteil anfällt, entweder in Lageenergie in einer Feder oder elektrische Energie in einer elektrischen Speichereinrichtung, beispielsweise in Gestalt eines Akkumulators, in einer aufladbaren Batterie oder in einem Kondensator zu speichern.

Eine separate Dämpfereinrichtung kann zwischen dem Oberteil und dem Unterteil angeordnet sein, um die Relativbewegung bei Unterstützung durch den Energiespeicher besser kontrollieren zu können. Durch die Überlagerung der Beeinflussung des Energiespeichers mit der separaten Dämpfereinrichtung kann eine präzisere und sichere Beeinflussung des Gangbildes erfolgen. Die Dämpfereinrichtung kann verstellbar ausgebildet sein, um in Abhängigkeit von Sensordaten, beispielsweise bezüglich des Gelenkwinkels, der Ganggeschwindigkeit, einer Winkelgeschwindigkeit oder eines Absolutwinkels eines Oberteils und/oder eines Oberteils eine angepasste Dämpfung bereitzustellen. Die Dämpfereinrichtung kann über einen Aktuator verstellt werden, um eine Verringerung oder Vergrößerung der Dämpfung zu erreichen.

Die Umwandlungseinrichtung kann verstellbar mit dem Oberteil und/oder mit dem Unterteil gekoppelt sein, um eine Eingriffsposition oder einen Verstellweg zu verlagern. Dadurch ist es möglich, sowohl die Energiemenge als auch den Zeitpunkt der Energiezufuhr wie gewünscht zu beeinflussen.

Mit dem beschriebenen Verfahren und der beschriebenen Einrichtung ist es möglich, dass benötigte Energie nur an geeigneter Stelle während des Bewegungsablaufes und in minimaler Menge zugeführt wird. Der Nutzer der orthopädietechnischen Einrichtung bringt weiterhin den größten Teil der Bewegungsenergie ein, beispielsweise über den verbliebenen Stumpf. Notwendig oder zumindest vorteilhaft ist eine Energiezufuhr jedoch an zwei Punkten, an denen der Nutzer der Gelenkeinrichtung über seine Muskeln die benötigte Energie nicht einbringen kann, beispielsweise weil die Muskelkraft nicht ausreicht, wenn wegen der Massenträgheiten sehr hohe Beschleunigungen erforderlich sind. So ist beispielsweise eine Unterstützung in der Schwungphase beim Überschwingen über den Boden sinnvoll, damit verhindert wird, dass das Unterteil beim Gehen auf dem Boden schleift. Über eine gezielte Energiezufuhr kann der Kniewinkel erhöht werden, sodass mehr Bodenfreiheit realisiert wird. Beim alternierenden Treppaufgehen kann ein zusätzliches Extensionsmoment den Benutzer befähigen, den Rest der Stufe aus eigener Kraft zu überwinden.

Aufgrund der Tatsache, dass immer nur die minimal nötigen Energien als kinetische Energie in das System eingespeist werden, behält der Nutzer jederzeit die Kontrolle über die orthopädietechnische Einrichtung. Die jeweils gespeicherte Energie würde nicht ausreichen, um den Benutzer in eine kritische Lage zu bringen. Darüber hinaus wird die Entstehung muskulärer Defizite vermieden, da der Benutzer weiterhin die Bewegungsenergie selbst aufbringen muss, lediglich die Feinsteuerung erfolgt über das Verfahren bzw. die Einrichtung.

Nachfolgend werden Ausführungsbeispiele der Erfindung anhand der beigefügten Figuren näher erläutert. Es zeigen:
- Figur 1 -: einen Kniewinkelverlauf während eines Gangzyklus;
- Figur 2 -: der Verlauf der Kniewinkelgeschwindigkeit über einen Gangzyklus;
- Figur 3 -: Phasen möglicher Energieaufnahmen und Abgaben des Kniegelenkes während des Gangzyklus;
- Figur 4 -: eine schematische Darstellung einer Gelenkeinrichtung mit einer Energiespeichereinrichtung zur Flexionsunterstützung;
- Figur 5 -: eine Gelenkeinrichtung mit einer Einrichtung zur Extensionsunterstützung;
- Figur 6 -: eine Gelenkeinrichtung mit einer in einer Kurvenbahn gelagerten Feder;
- Figur 7 -: eine Gelenkeinrichtung mit einer Schwungmasse als Energiespeicher;
- Figur 8 -: eine Variante der Gelenkeinrichtung mit einer Extensionssteuerung;
- Figur 9 -: eine Gelenkeinrichtung mit zwei Federn
- Figur 10: eine Gelenkeinrichtung mit einer elastischen Sehne als Energiespeicher;
- Figur 11: eine Variante der Figur 1 mit einer verschieblichen Federanbindung;
- Figur 12: eine Variante mit einer im Unterteil angeordneten Feder;
- Figur 13: eine Variante der Figur 3 mit einem zwischengeschalteten Aktuator;
- Figur 14: eine Variante mit einem Drillfaden als Energiespeicher;
- Figur 15: eine Darstellung von Antriebsmomentverläufen bei unterschiedlichen Gehgeschwindigkeiten;
- Figur 16: eine Darstellung unterschiedlicher Verlagerungswege über einen Gelenkwinkel bei verschiedenen Gehgeschwindigkeiten;
- Figur 17: eine Darstellung eines Hebelarmverlaufes über einen Gelenkwinkel; sowie
- Figur 18: eine Darstellung einer Flexionsdämpfungseinstellung eines Dämpfers über einen Kniewinkel.

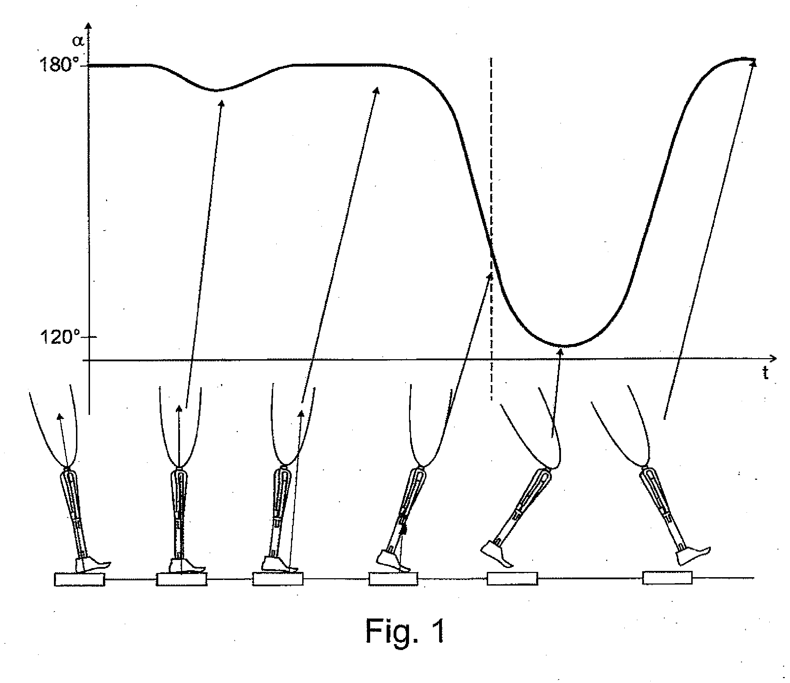

In der Figur 1 ist eine schematische Darstellung des Verlaufes des Kniewinkels α über der Zeit dargestellt. Die Darstellung umfasst einen Gangzyklus, also beginnend von dem Aufsetzen der Ferse bis zum erneuten Aufsetzen der Ferse desselben Beines. Nach dem initialen Fersenauftritt mit einem gestreckten Kniegelenk, also bei einem Kniewinkel α von 180°, beugt das Kniegelenk in der Standphase zunächst ein wenig ein, was als Standphasenflexion bezeichnet wird. Sobald der Fuß vollständig auf den Boden aufsetzt, wird das Kniegelenk gestreckt, so dass sich ein Kniewinkel α von 180° einstellt. Im Verlauf der Beugebewegung gegen Ende der Standphase nimmt der Kniewinkel α ab. Die senkrechte, gestrichelte Linie bezeichnet das Ende der Standphase und damit den Zeitpunkt, an dem die Fußspitze den Boden verlässt. Dieser Zeitpunkt wird "toe off" genannt. In der sich anschließenden Schwungphase schwingt das Unterteil weiter nach hinten und verschwenkt relativ zum Oberteil bis zum minimalen Kniewinkel α von 120°. Anschließend erfolgt eine Bewegungsumkehr, der Unterschenkel wird mit dem Prothesenfuß nach vorne gebracht und verschwenkt bis in den Streckanschlag, der bei einem Kniewinkel α von 180° liegt. In dieser gestreckten Stellung wird in der Regel der Fersenauftritt erfolgen.

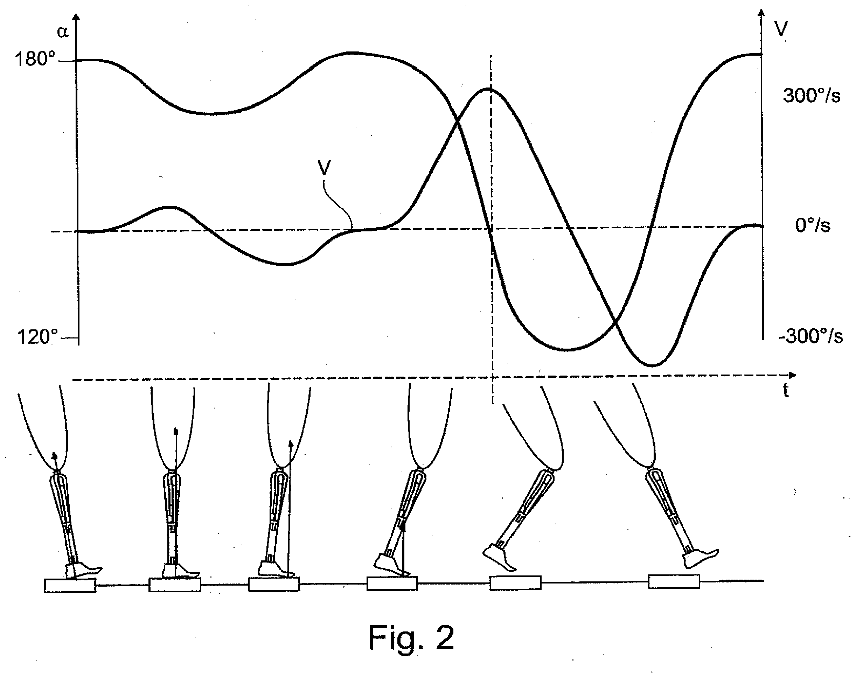

In der Figur 2 ist eine schematische Darstellung des Kniewinkels α mit der Kniewinkelgeschwindigkeit v überlagert. Der Kniewinkelverlauf entspricht dabei dem in der Figur 1 beschriebenen. Während der anfänglichen Standphasenflexion nimmt die Kniewinkelgeschwindigkeit v kurzzeitig zu. Während der gestreckten Phase, also bei einem Kniewinkel α von 180°, verändert sich der Kniewinkel nicht, ebenfalls bleibt die Kniewinkelgeschwindigkeit v konstant bei 0° pro Sekunde. Das Einbeugen des Kniegelenkes zum Ende der Standphase führt zu einem Anstieg der Kniewinkelgeschwindigkeit v bis zu einem Maximum beim "toe off". Die weitere Rückschwungbewegung des Unterteils findet mit einer sich verlangsamenden Geschwindigkeit statt, bis bei einem minimalen Kniewinkel α eine Bewegungsumkehr einsetzt und das Unterteil eine Extensionsbewegung ausführt. Dementsprechend verläuft die Kniewinkelgeschwindigkeit unterhalb der Nulllinie bis zu dem Zeitpunkt, in dem eine Extensionsdämpfung einsetzt, um das Unterteil nicht ungebremst in den Streckanschlag schwenken zu lassen. Dementsprechend verringert sich die Kniewinkelgeschwindigkeit v, bis das Unterteil den Streckanschlag erreicht hat und der Kniewinkel α wieder 180° beträgt. Die Kniewinkelgeschwindigkeit v beträgt dann 0.

Um schneller in den Bereich einer großen Kniebeugung zu gelangen, ist es möglich und notwendig, in bestimmten Abschnitten des Gangzyklus die Kniewinkelgeschwindigkeit zu erhöhen, beispielsweise um damit ein Durchschwingen des Beines zu erleichtern. Die maximale Kniebeugung kann dabei gleich bleiben oder aber vergrößert werden, wenn eine Extensionsunterstützung vorhanden ist, damit der Unterschenkel schnell genug nach vorne gebracht wird.

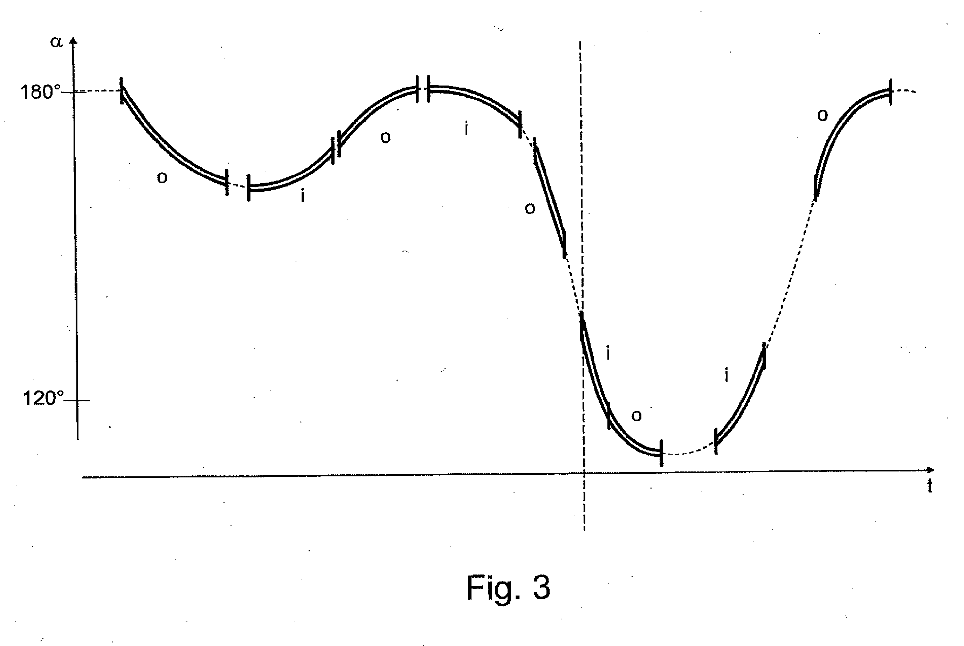

In der Figur 3 ist erneut der Kniewinkel α über der Zeit während eines Gangzyklus dargestellt. Darin sind Bereiche markiert, in denen Bewegungsenergie aus der Relativbewegung zwischen dem Unterteil und dem Oberteil umgewandelt oder gespeichert werden kann, diese Bereiche sind mit dem Bezugszeichen o versehen. Weiterhin sind Bereiche mit dem Bezugszeichen i markiert, in denen es vorteilhaft sein kann, gespeicherte Energie dem System erneut zuzuführen, um eine Extension oder Flexion zu unterstützen. Während der anfänglichen Standphasenbeugung kann Bewegungsenergie aufgenommen werden, um diese beispielsweise in der unmittelbar darauf folgenden Extensionsphase wieder zuzuführen. Ebenfalls ist es möglich, die aufgenommene Energie in der Beugephase oder aber auch zum Ende der Streckphase zwischenzuspeichern und erst während einer weiteren Energieaufnahmephase erneut zuzuführen, so dass das Unterteil beispielsweise schneller flektiert oder extendiert wird, um den jeweils gewünschten Effekt zu erreichen. Die gesteuert zeitversetzte Zuführung der umgewandelten oder gespeicherten Energie erfolgt innerhalb desselben Bewegungszyklus während vorbestimmter Phasen i, wobei bevorzugt Bremsphasen genutzt werden, um Energie umzuwandeln und/oder zu speichern und Beschleunigungsphasen, um diese Energie wieder gesteuert zeitversetzt zuzuführen. Die Steuerung erfolgt bevorzugt elektronisch, grundsätzlich ist jedoch auch eine mechanische Steuerung möglich.

Als Zuführphasen i sind insbesondere die Extensionsbewegung nach dem initialen Fersenkontakt, die Unterstützung der Einbeugung zur Einleitung der Schwungphase, die Aufrechterhaltung der Flexionsgeschwindigkeit nach dem "toe off" sowie die Unterstützung der Flexionsbewegung nach dem Erreichen des maximalen Flexionswinkels vorgesehen.

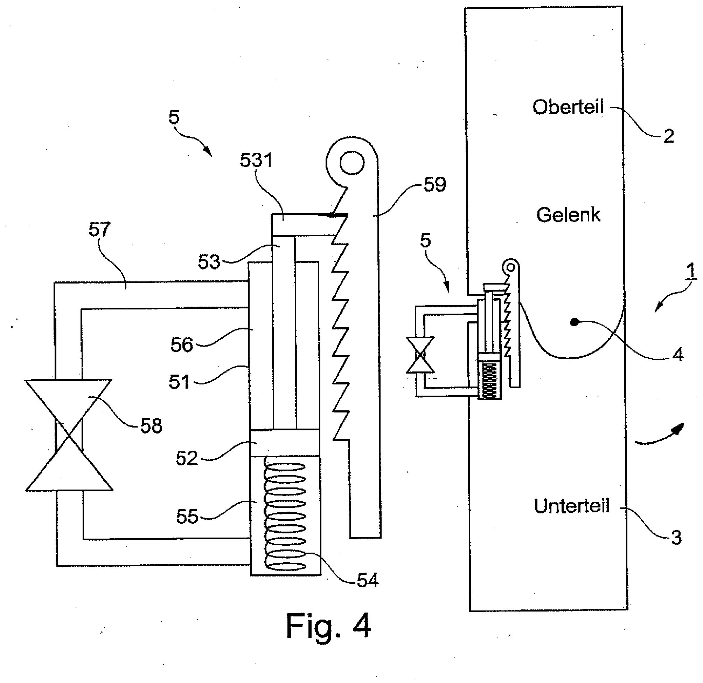

In der Figur 4 ist ein Beispiel einer orthopädietechnischen Gelenkeinrichtung 1 in einer schematischen Darstellung gezeigt. Die orthopädietechnische Gelenkeinrichtung 1 weist ein Oberteil 2 und ein Unterteil 3 auf. Das Unterteil 3 ist um eine Schwenkachse 4 drehbar an dem Oberteil 2 gelagert. Die Flexion erfolgt in der Pfeilrichtung, eine Energieumwandlungseinrichtung 5 ist auf der Extensionsseite angeordnet. Die Energieumwandlungseinrichtung 5 weist einen Hydraulikzylinder 51 auf, in dem ein Kolben 52 an einer Kolbenstange 53 angeordnet ist. Über den Kolben 52 werden zwei Kammern 55, 56 hydraulisch voneinander getrennt. In der unteren Kammer 55 ist eine Druckfeder angeordnet. Beide Kammern 55, 56 sind über eine Hydraulikleitung 57 miteinander gekoppelt. In der Hydraulikleitung 57 ist ein steuerbares Ventil 58 angeordnet, über das die Durchflussrate von der oberen Kammer 56 in die untere Kammer 55 gesteuert werden kann. An dem oberen Ende der Kolbenstange 53 ist ein sägezahnartig abgeschrägtes Formschlusselement 531 angeordnet, das in Eingriff mit einer schwenkbar gelagerten Zahnstange 59 steht, die ebenfalls eine sägezahnförmige Verzahnung aufweist, die korrespondierend zu der des Formschlusselementes 531 ausgebildet ist. Bei einer Extensionsbewegung des Unterteils 3 relativ zu dem Oberteil wird der Kolben 52 eingeschoben. Um eine Flexionsunterstützung einzuleiten, wird das Ventil 58 so weit geöffnet, dass das Ventil 58 eine schnellere Kolbenbewegung zulässt als die Gelenkeinrichtung. Zur Spannung der Feder 54 wird die Kolbenstange 53 nach unten gedrückt. Dies geschieht während einer Extensionsbewegung des Unterteils 53 relativ zu dem Oberteil. Flektiert das Unterteil 3 relativ zu dem Oberteil 2, gleiten die Zähne der Zahnstange 53 aufgrund ihrer Orientierung an der Schräge des Formschlusselementes 531 entlang, bei der Extension des Unterteils 3 wird die Feder 54 komprimiert, da durch die im wesentlichen waagerechten Kontaktflächen ein Abgleiten der Zahnstange 59 nicht möglich ist. Zur Flexionsunterstützung, beispielsweise bei der Einleitung der Schwungphase, also bei einem relativ stark gebeugten Unterteil 3, steht das Formschlusselement 531 relativ weit unten an der Zahnstange 59 in Eingriff und unterstützt die Bewegung des Unterteils 3 in Pfeilrichtung. Zur Flexionsunterstützung wird das Ventil 58 mechanisch oder elektrisch in Abhängigkeit von einer ermittelten Gelenkwinkelposition oder in Abhängigkeit von einer Gelenkwinkelgeschwindigkeit mechanisch oder elektrisch gesteuert. Wird das Ventil 58 geöffnet, kann sich die Feder 54 entspannen, da aus der oberen Kolbenkammer 56 durch die Hydraulikleitung 57 das Hydraulikfluid in die untere Kammer 55 strömen kann.

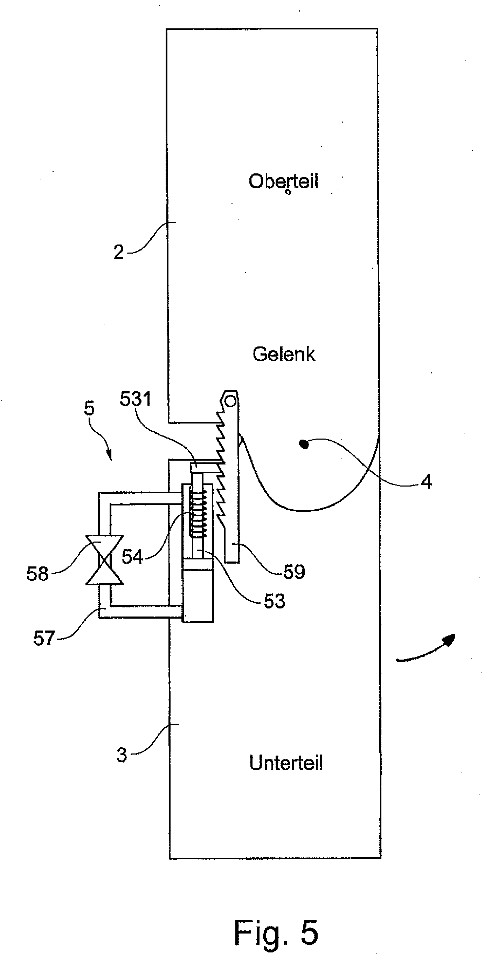

In der Figur 5 ist eine Gelenkeinrichtung gezeigt, bei der die Wirkrichtung im Vergleich zu der Ausführung gemäß Figur 4 umgedreht ist. Die Feder 54 ist um die Kolbenstange 53 herum angeordnet, das Formschlusselement 531 ist ebenso wie die Anordnung der Zähne in der Zahnstange 59 in die entgegengesetzte Richtung orientiert, so dass eine Extensionsunterstützung vorliegt. Der Kolben 52 wird bei einer Flexion herausgezogen und wirkt unterstützend für die Extension, wenn das Ventil 58 eine Kolbenbewegung zulässt, die die Feder 54 unterstützt. Dies kann insbesondere bei der Umkehrung der Flexionsbewegung in eine Extensionsbewegung erfolgen. Zur Steuerung der Unterstützung der Extensionsbewegung kann die Zahnstange 59 oder Ratsche mechanisch ausgekoppelt werden oder das Ventil 58 dämpfend eingreifen.

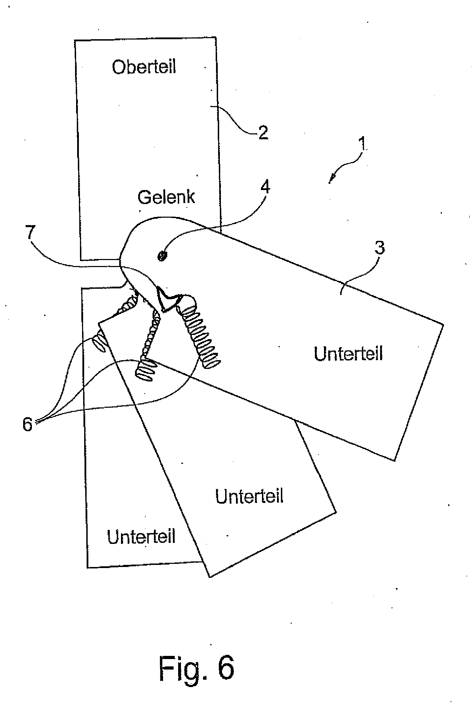

In der Figur 6 ist eine weitere Variante der Erfindung dargestellt, bei der bei der Flexion des Gelenkes eine Feder 6 über eine Kurvenbahn 7 komprimiert wird, um bei einer weiteren Beugung die Energie wieder abzugeben. Somit kann kinetische Energie während einer Flexionsbewegung, beispielsweise während des Fersenstoßes, gespeichert werden und zur Einleitung der Schwungphase zur Unterstützung der Flexionsbewegung und/oder zur Aufrechterhaltung der Beugegeschwindigkeit nach dem "toe off' wieder der Gelenkeinrichtung 1 zugeführt werden. Bei der Extensionsbewegung bleibt die Feder 6 aufgrund der Führung in der Kurvenbahn 7 wirkungslos.

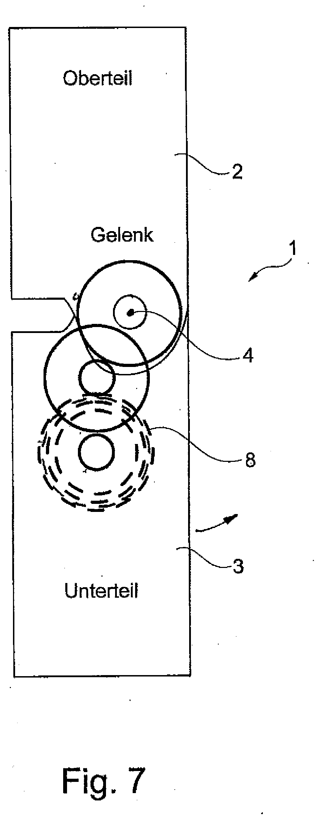

Eine weitere Variante der Gelenkeinrichtung 1 ist in der Figur 7 dargestellt. Dabei ist als Einrichtung zur Umwandlung und Speicherung kinetischer Energie eine Schwungmasse 8 vorgesehen, die bei der Flexion des Unterteils 3 relativ zu dem Oberteil 2 beschleunigt wird. Bei der Verlangsamung der Flexionsgeschwindigkeit gibt die Schwungmasse 8 die Rotationsenergie wieder ab, so dass nach dem Erreichen der maximalen Flexionsgeschwindigkeit, also nach dem "toe off" oder dem Durchschwingen in der Extensionsphase zur Unterstützung der jeweiligen Bewegung Energie der Gelenkeinrichtung 1 zugeführt wird.

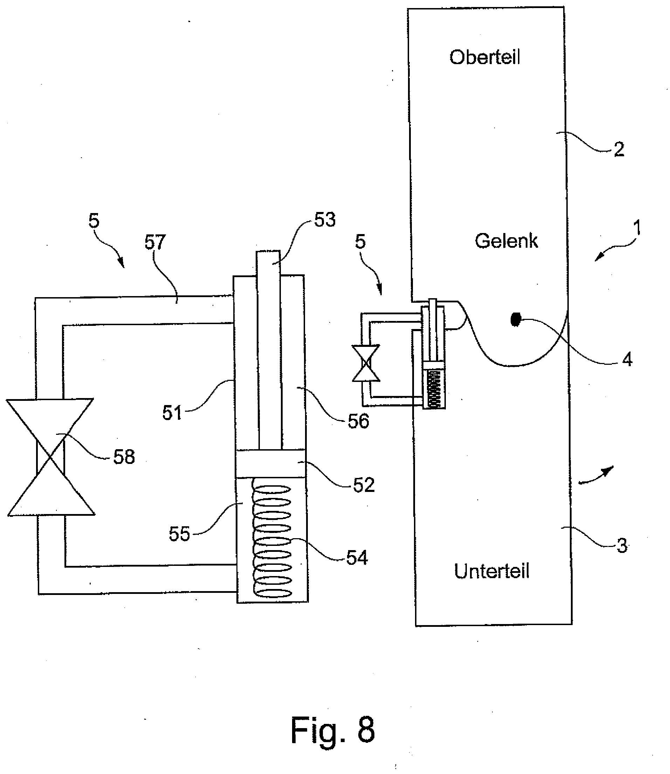

In der Figur 8 ist die Einrichtung 5 zur Umwandlung und Speicherung kinetischer Energie ähnlich wie in der Figur 4 beschrieben aufgebaut, es fehlen jedoch das Formschlusselement 531 am oberen Ende der Kolbenstange 53 sowie die Zahnstange 59. Mit einer solchen Vorrichtung ist es möglich, eine Extensionssteuerung zu betreiben, insbesondere den Extensionsanschlag einzustellen. Wird die Gelenkeinrichtung vollständig gestreckt, wird der Kolben 52 maximal in die untere Kammer 55 hinein bewegt. Die Druckfeder 54 ist maximal gespannt. Die so gespeicherte Energie kann beim Öffnen des Ventils 58 zur Flexionsunterstützung freigegeben werden, so dass zur Flexionseinleitung am Ende der Standphase eine Unterstützung erfolgt. Je weiter die Kolbenstange 53 dabei ausgefahren wird, desto größer ist der Kniewinkel a, in dem die Extensionssteuerung wirkt, da das obere Ende der Kolbenstange 53 oder ein ihr zugeordnetes Bauteil frühzeitig mit der Anschlagfläche im Oberteil 2 in Kontakt tritt.

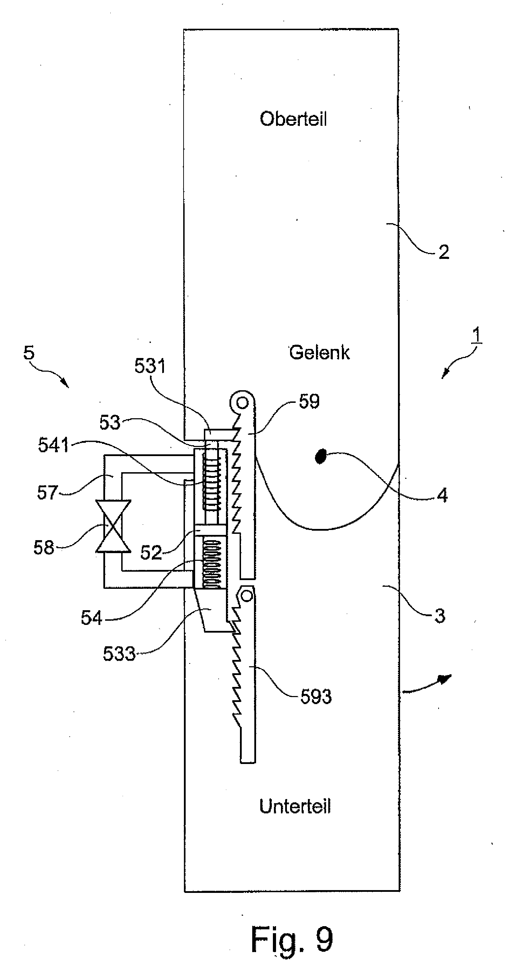

In der Figur 9 ist eine weitere Variante der Erfindung dargestellt. Die Energieumwandlungseinrichtung 5 sieht zusätzlich zu der Ausgestaltung gemäß Figur 4 eine zweite Zahnstange 593 und ein zweites Formschlusselement 533 vor. Die zweite Zahnstange 593 ist an dem Unterteil 3 schwenkbar angeordnet, das zweite Formschlusselement 533 wirkt auf die Feder 54 in der unteren Kolbenkammer 55. Der Kolben 52 mit der Kolbenstange 53 wird bei der Extension über die Zahnstange 59 eingeschoben. Die Feder 54 speichert die kinetische Energie als Lageenergie. Je nach beabsichtigter Unterstützung wird entweder die kolbenstangenseitige Feder 541 oder die kolbenstangenferne Feder 54 komprimiert.

Der Energieumwandlungseinrichtung 5 kann eine geschwindigkeitsabhängige Kupplung zugeordnet sein, die bei erhöhten Kniewinkelgeschwindigkeiten v eine verminderte Reibung zwischen Kraftübertragungselementen bereitstellt, so dass die Umwandlungs- oder Speicherungsrate umgekehrt proportional zu der Verschwenkgeschwindigkeit des Unterteils 3 relativ zu dem Oberteil 2 ist.

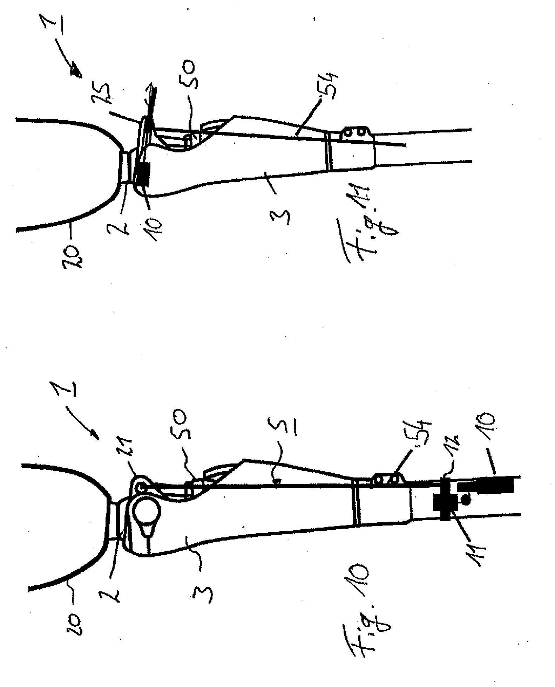

Figur 10 zeigt eine orthopädietechnische Gelenkeinrichtung in Gestalt eines Prothesenkniegelenkes mit einem Oberteil 2, an dem ein Oberschenkelschaft 20 zur Aufnahme eines Oberschenkelstumpfes angeordnet ist. Distal zu dem Oberteil 2 ist ein Unterteil 3 gelenkig befestigt, so dass das Oberteil 2 relativ zu einem Unterteil 3 verschwenkt werden kann. An dem Oberteil 2 ist rückwärtig ein Ausleger 21 ausgebildet, an dem einerseits eine Dämpfereinrichtung 50 in Gestalt eines hydraulischen oder pneumatischen Dämpfers und andererseits ein Energiespeicher 54 in Gestalt einer elastischen Sehne angeordnet ist. Die elastische Sehne ist über ein Übersetzungsgetriebe 11 mit einem Aktuator 10 in Gestalt eines Elektromotors verbunden. Der Elektromotor ist in einem Unterschenkelrohr angeordnet, das an dem Unterteil 3 befestigt ist. Der Energiespeicher 5 in Gestalt der elastischen Sehne ist an dem Übersetzungsgetriebe 11 und einem Ausleger 12 befestigt, wird der Motor 10 aktiviert, wirkt dieser über das Übersetzungsgetriebe 11 auf den Ausleger 12 ein und kann die elastische Sehne 54 entweder spannen oder entspannen, indem der Ausleger 12 in distale oder proximale Richtung verlagert oder in die eine oder andere Richtung verdreht wird, um die elastische Sehne auf- oder abzurollen. Der Ausleger 12 bildet somit einen verlagerbaren Lagerungspunkt des Energiespeichers 5, wodurch es möglich ist, bei einer Extensionsbewegung des Unterteils 3 den Beginn eines Spannvorganges der elastischen Sehne 54 einzustellen. Über den Ausleger 12 kann ein verlagerbarer, elastischer Streckanschlag realisiert werden, der über den Aktuator 10 verstellt wird. Über eine Extensionsbewegung des Unterteils 10 wird der als Feder ausgebildete Energiespeicher 54 gespannt und nimmt einen Teil der Bewegungsenergie des Unterteils 3 auf. Dies kann beispielsweise am Ende der Schwungphase oder nach dem Fersenstoß und der Standphasenflexion erfolgen. Im Rahmen der Standphasenextension wird die Feder 54 gespannt und während der Standphase weiter gespannt beibehalten.

In der terminalen Standphase kann zur Unterstützung der Schwungphaseneinleitung die gespeicherte Energie wieder abgegeben werden, die elastische Sehne 54 zieht sich zusammen und wandelt die Lageenergie in mechanische Arbeit um, um die Flexion des Unterteils 3 zu unterstützen. Soll mehr Energie in dem Energiespeicher 54 gespeichert werden, spannt der Aktuator 10 die elastische Sehne 54 vor, indem der Ausleger 12 distal verlagert oder in Aufrollrichtung verdreht wird, soll weniger Energie gespeichert werden, wird der Ausleger 12 proximal verlagert oder die Sehne abgerollt. Im dargestellten Ausführungsbeispiel ist die Energiespeichereinrichtung 54 gleichzeitig die Umwandlungseinrichtung 5, in der die mechanische Arbeit aus der Relativbewegung in potenzielle Energie umgewandelt wird.

Zusätzlich zu der Umwandlungseinrichtung 5 bzw. dem Energiespeicher 54 ist ein separater Dämpfer 50 in Gestalt eines Hydraulik- oder Pneumatikdämpfers vorgesehen, der verstellbar ausgebildet ist, so dass über die Dämpfereinrichtung 50 die Dämpfung während des Gehens sowohl in Flexionsrichtung als auch Extensionsrichtung beeinflusst werden kann.

Zur kontrollierten Unterstützung bei der Schwungphaseneinleitung ist es vorgesehen, dass über den Aktuator 10, die Übersetzung 11 und die Verlagerung oder Verdrehung des Auslegers 12 eine Veränderung der Vorspannung der elastischen Sehne 54 erfolgt, um die Energieabgabe besser kontrollieren zu können. Es hat sich herausgestellt, dass eine Feder alleine als Kraftspeicher eine zu große und zu schnelle Krafteinleitung bewirkt, was von den Patienten als unangenehm empfunden werden kann. Um sowohl den Zeitraum der Energieeinleitung als auch die Energiemenge und die Leistung kontrollieren zu können, kann in Abhängigkeit von der Winkelstellung des Oberteils 2 zum Unterteil 3, der Winkelstellung des Oberteils 2 und/oder des Unterteils 3 zueinander oder im Raum, den Winkelgeschwindigkeiten oder der Ganggeschwindigkeit eine Manipulation am Energiespeicher 54 vorgenommen werden, um die Leistung zu begrenzen und darüber hinaus den zeitlichen Ablauf der Energieabgabe zu steuern. Durch Entspannen der Feder 54 ist es möglich, weniger Energie in die Gelenkeinrichtung 1 einzubringen, durch Nachspannen der Feder 54 ist es möglich, eine Unterstützung der Flexion über einen längeren Zeitraum hinweg und über einen größeren Flexionswinkel hinweg aufrechtzuerhalten, um das gewünschte harmonische Gangbild zu erreichen.

Eine Variante der Erfindung ist in der Figur 11 dargestellt, bei der statt der Entspannung oder Spannung der Feder 54 im Wesentlichen in ihrer Längserstreckung eine Verlagerung an dem distalen Lagerungspunkt erfolgt. Der obere Befestigungspunkt ist in einer verschieblichen Federanbindung 25 geführt, die über den Aktuator 10 in Richtung des Doppelpfeils hin- und herschiebbar ist. Je nach Anlenkpunkt und Bewegungsrichtung wird die elastische Sehne 54 gespannt oder entspannt. Sowohl in der Figur 10 als auch in der Figur 11 wird die Energie aus der Extensionsbewegung in der elastischen Sehne 54 gespeichert. Nach Beendigung der Extensionsbewegung ist es möglich, dass der Motor 10 die Sehne 54 anschließend nachspannen kann, wenn die erwartete aufzubringende Energie nicht ausreicht, um eine gewünschte Unterstützung bei der Flexionseinleitung zu bewirken. Das Nachspannen erfolgt vorteilhafterweise dann, wenn sich die Gelenkeinrichtung 1 in einem vollständig extendierten Zustand befindet, um möglichst wenig gegen eine Vorspannungbewegung anarbeiten zu müssen. Über die Verstellung entweder der Vorspannung der elastischen Sehne 54 oder der proximalen Lagerungsposition ist es möglich, den Streckwinkel einzustellen, ab dem die Umwandlungseinrichtung 5 aktiv wird, worüber sich auch einstellen lässt, wie viel Energie in dem Energiespeicher 5 gespeichert werden soll.

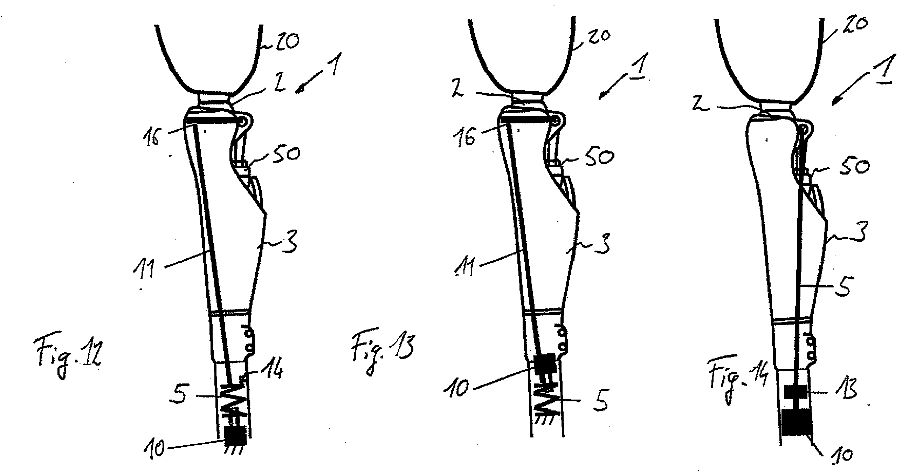

In der Figur 12 ist eine Variante der Gelenkeinrichtung 1 dargestellt, bei der die Umwandlungseinrichtung 5 in Gestalt der Feder 54 im Unterschenkelrohr angeordnet ist. Der Aktuator 10 ist mit der Feder 5 verbunden und kann diese entweder komprimieren oder aufwickeln, je nach Ausgestaltung der Feder als Druckfeder oder Spiralfeder. Die Feder 5 ist über eine Druckstange 11 mit einem Anschlag 16 gekoppelt, der an dem Oberteil 2 befestigt ist. Durch eine Aktivierung des Motors 10 kann der Fußpunkt der Feder 5 verändert werden, wodurch sich über die Druckstange 11 einstellen lässt, wann die Feder 5 mit dem Anschlag 16 in Kontakt kommt. Je früher die Druckstange 11 mit dem Anschlag 16 in Kontakt kommt, desto größer ist der Verstellweg und die Kompression der Feder 5, so dass entsprechend mehr Energie in der Feder 5 gespeichert wird. Dementsprechend wird bei einer Rückumwandlung mehr Energie aus der Feder über die Druckstange 11 auf den Anschlag 16 übertragen, so dass eine vergrößerte Flexionsunterstützung erreicht werden kann. Um die Energieabgabe zu beeinflussen, wird die Feder 5 bei der Bewegungsunterstützung entweder gespannt oder entspannt.

Eine Variante ist in der Figur 13 dargestellt, die im Wesentlichen der Figur 12 entspricht, jedoch ist der Aktuator 10 gegebenenfalls mit Spindeltrieb und einem Freilauf in eine Richtung zwischen der Feder 5 und der Druckstange 10 angeordnet, so dass der Fußpunkt der Feder 5 fest bleibt, sich jedoch die Feder 5 mit dem Motor 10 vorspannen lässt. Wird eine Flexionsunterstützung eingeleitet, muss der Motor 10 mitdrehen, um die Energie freizugeben und über die Druckstange 11 und den Anschlag 16 auf die Gelenkeinrichtung zu übertragen, wodurch sich eine besonders gute Kontrolle über die Energiefreigabe erreichen lässt. Ebenso ist es möglich, die Energiefreigabe zu stoppen, was sinnvoll sein kann, wenn eine Situation falsch eingeschätzt wurde.

In der Figur 14 ist ein Drillfaden als Energiespeicher 54 und Umwandlungseinrichtung 5 gezeigt, bei der über eine Verdrillung von Fäden eine Verkürzung erreicht wird. Durch Zunahme oder Abnahme der Verdrillung kann der Kontaktpunkt eingestellt werden, ab dem der Drillfaden Zugkräfte aufbaut. Zwischen dem Motor 10 und dem Drillfaden 54 ist eine Axialentkopplung 13 vorgesehen.

Neben der dargestellten Ausführungsform des Energiespeichers als Feder kann dieser auch gegebenenfalls über ein Übersetzungsgetriebe und einen Generator als elektrischer Energiespeicher in Gestalt einer Batterie, eines Akkumulators oder eines Kondensators ausgebildet sein. Zur Rückumwandlung der gespeicherten elektrischen Energie wird der Generator als Motor geschaltet, so dass ein Antrieb und eine Unterstützung der Relativverlagerung des Unterteils 3 zu dem Oberteil 2 erfolgen können. Zur Erhöhung der Energiemenge kann ein Generator dem elektrischen Energiespeicher zugeordnet sein, ebenso ist es möglich, einen weiteren Energiespeicher vorzusehen, der als Puffer dient, in den überschüssige elektrische Energie eingespeichert oder aus dem zusätzlich benötigte Energie bereitgestellt wird.

Die Federn als Energiespeicher 54 können als Zugfedern, Druckfedern, Drehfedern oder Elastomerelemente ausgebildet sein, die ab einem bestimmten Streckwinkel, der von dem Aktuator 10 eingestellt wird, in Kontakt kommen und sowohl mechanische Arbeit in Energie ab diesem Zeitpunkt umwandeln als auch wieder zur Bewegungsunterstützung zurückführen. Die Feder nimmt dabei die Energie aus der Bewegung in Extensionsrichtung auf, und dient gleichzeitig als Bremseinrichtung und Extensionsanschlag. Bei der Schwungphaseneinleitung wird die Energie wieder abgegeben und hilft dem Nutzer, die Schwungphase einzuleiten. Über den Aktuator 10 kann der Zeitpunkt des Kontaktes der Feder bei der Energieabgabe verstellt werden, so dass unterschiedliche, kontrollierte Unterstützungen bei verschiedenen Gehgeschwindigkeiten möglich sind. Ebenso ist es möglich, dass über den Motor 10 die jeweilige Feder nachgespannt wird, falls die durch die vorhergehende Bewegung gespeicherter Energie nicht ausreicht, um eine ausreichende Unterstützung bereitzustellen, beispielsweise kann beim besonders langsamen Gehen oder beim Treppabgehen die mechanische Arbeit nicht ausreichen, um die Feder ausreichend zu spannen. Der Feder 5 kann, wie in der Figur 12 gezeigt, eine Freigabeeinrichtung 14 zugeordnet sein, über die der Auslösezeitpunkt zur Freigabe der gespeicherten Energie zusätzlich abgesichert werden kann.

Um das Auslösen der Freigabe abzusichern, kann die Gelenkeinrichtung 1 eine Sicherung enthalten, die durch die Hydraulik in dem Dämpfer 50 oder durch eine Steuerung des Motors 10 ausgebildet wird, die sicherstellen, dass die aufgebrachte Federenergie rechtzeitig wieder abgebaut wird.

Aufgrund der Tatsache, dass die Bewegungsenergie bei der Extension zumindest teilweise gespeichert wird, kann die motorische Unterstützung sehr sparsam arbeiten. Die Batterie für den Aktuator 10 kann klein und leicht ausfallen, ebenso wie der Aktuator 10 selbst, da der Aktuator 10 beim Nachspannen in der Standphase ausreichend Zeit hat, die Feder zu spannen und das Einspeisen der Energie nicht so schnell erfolgen muss, wie es die Abgabe für die Schwungphaseneinleitung erfordert. Der Motor 10 steuert die Energiefreigabe aus der Feder, gegebenenfalls in Verbindung mit den separaten Dämpfer 20. Die Flexionsunterstützung durch den Energiespeicher hilft beim Erreichen des notwendigen Beugewinkels beim alternierenden Treppensteigen sowie beim Übersteigen von Hindernissen und erspart Hüftarbeit.

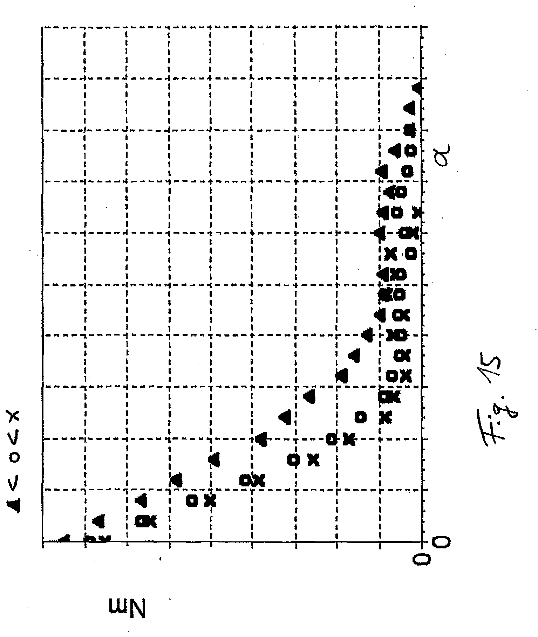

In der Figur 15 ist beispielhaft das Antriebsmoment bei verschiedenen Gehgeschwindigkeiten über den Gelenkwinkel α dargestellt. Die Darstellung erfolgt für drei unterschiedliche Gehgeschwindigkeiten, wobei die jeweiligen Gehgeschwindigkeiten durch unterschiedliche Symbole in dem Diagramm dargestellt werden, die niedrigste Gehgeschwindigkeit ist mit einem Dreieck gekennzeichnet, die mittlere durch einen Kreis und die höchste Gehgeschwindigkeit durch ein X. Das Antriebsmoment ist das effektive Antriebsmoment in Nm, also die durch die Speichereinrichtung 5 gespeicherte und wieder zugeführte Energie abzüglich der Verluste wie Dämpfung oder Reibung. Es ist zu erkennen, dass anfänglich mit einem sehr hohen Antriebsmoment gearbeitet wird, um die Flexionsunterstützung zu Beginn bereitstellen zu können. Bei zunehmendem Gelenkwinkel a, vorliegend dem Kniewinkel, der von einer maximalen Extensionsstellung aus gemessen wird, fällt das aufzubringende Antriebsmoment zunächst steil ab, bleibt über einen kleinen Winkelbereich konstant, steigt kurz wieder an und fällt dann bis zur maximalen Flexion auf null ab. Es ist zu erkennen, dass die Flexionsunterstützung bei geringen Gehgeschwindigkeiten, repräsentiert durch das Dreieck, größer ausfällt als bei hohen Gehgeschwindigkeiten. Der Antriebsmomentverlauf, wie er in der Figur 15 zeigt ist, ist mit einer Feder ohne einen motorischen Einfluss durch Nachspannen oder Entspannen nicht erzeugbar, da erfindungsgemäß nach dem starken Rückgang des Antriebsmomentes über einen weiteren Zeitraum das Moment bis zum Erreichen des Maximalwinkels aufrechterhalten wird.

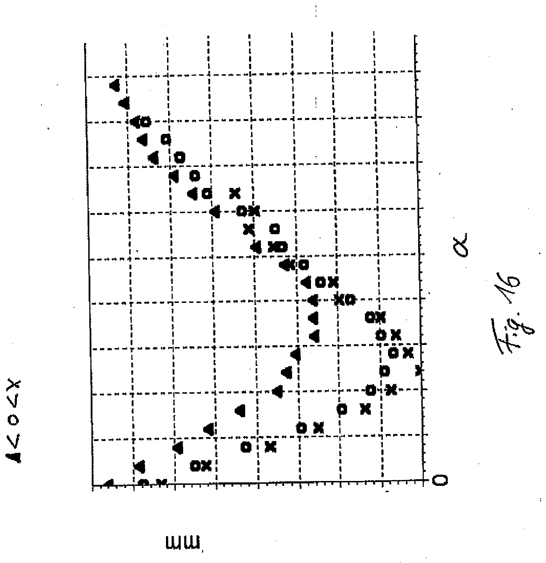

Figur 16 zeigt ein Diagramm, bei dem der Zugweg des Motors 10 über den Gelenkwinkel α aufgetragen ist. Es sind verschiedene Gehgeschwindigkeiten aufgeführt, die wiederum durch ein Dreieck, einen Kreis und ein X gekennzeichnet sind, die geringste Gehgeschwindigkeit ist durch ein Dreieck repräsentiert. Die Darstellung bezieht sich auf die translatorische Bewegung des Motors 10 bei den Ausführungsformen der Figuren 10, 12 und 14. Der Verlagerungsweg gemäß Figur 16 ist so abgestimmt, dass die Antriebsmomentkurve gemäß Figur 15 erreicht werden kann. Der jeweilige Verlauf ist für jede gewählte Feder verschieden und kann je nach Eigenschaft der Feder zu einem größeren oder kleineren Verlagerungsweg führen. Ziel ist es, eine möglichst geringe Verlagerung und damit Spannung der Feder zu erreichen. Beim Start der Beugeeinleitung ist zu erkennen, dass der Motor die Feder zurückgleiten lässt, um eine möglichst schnelle Kraftreduzierung zu erreichen, damit das Gefühl einer kontrollierten Flexion weiter aufrechterhalten bleibt. Bei größeren Winkeln wird die Feder wieder gespannt, um die Kraft aufrechtzuerhalten bzw. wieder zu erhöhen. Auch hier ist es signifikant, dass bei langsamen Gehgeschwindigkeiten eine vergrößerte Unterstützung notwendig ist. Die Freigabe der Feder und damit der Energie zur Flexionsunterstützung und der Antrieb des Motors erfolgen gleichzeitig, so dass über den gesamten Verlauf der Flexionsunterstützung eine Kontrolle möglich ist.

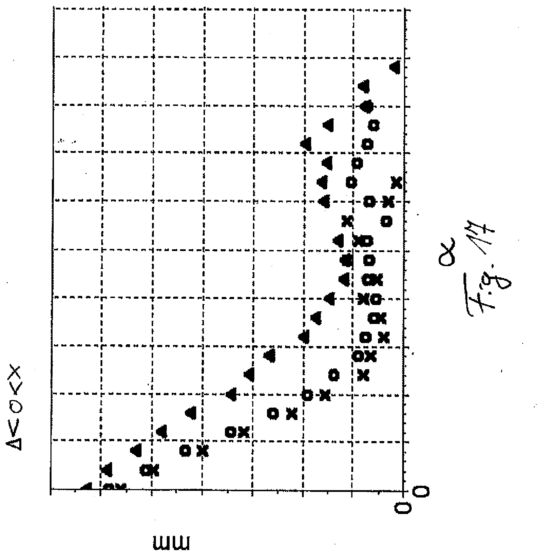

Die Figur 17 zeigt die Veränderung des Hebelarms gemäß einer Ausführungsform der Figur 11 über den Gelenkwinkel α für unterschiedliche Gehgeschwindigkeiten. Auch hier wird gleichzeitig mit der Federfreigabe der Motor 10 aktiviert, um den Hebelarm zu verstellen. Zunächst wird der Hebelarm schnell verkleinert, um einen Kraftabbau zu bewirken, anschließend wird der Hebelarm wieder vergrößert, um über einen größeren Winkelbereich α Kraft aufzubringen und die Flexionsbewegung zu unterstützen.

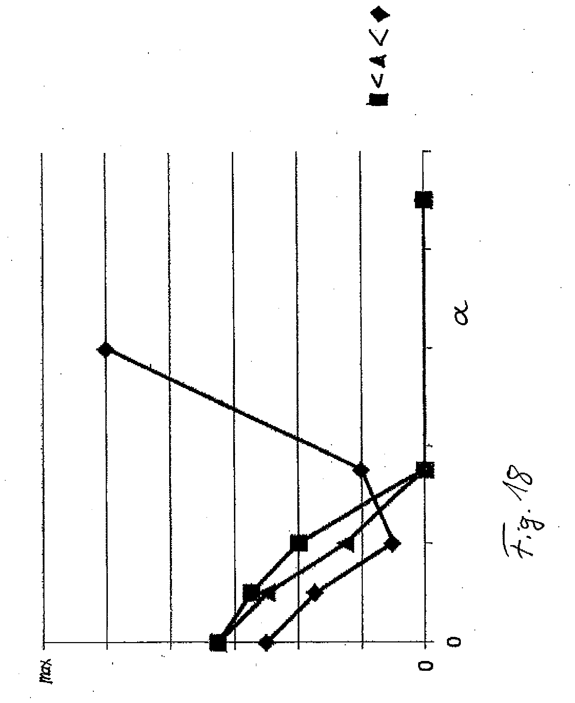

In der Figur 18 ist die Flexionsdämpfungseinstellung der Dämpfereinrichtung 50 über den Gelenkwinkel α bei verschiedenen Geschwindigkeiten dargestellt. Abweichend zu den vorherigen Figuren ist die niedrigste Gehgeschwindigkeit durch ein Quadrat gekennzeichnet, die mittlere durch ein Dreieck und die höchste Gehgeschwindigkeit durch eine Raute. Zunächst ist eine mittlere Flexionsdämpfungseinstellung der Dämpfereinheit 50 vorgesehen, die mit zunehmendem Gelenkwinkel α abnimmt. Bei hohen Gehgeschwindigkeiten kann zum Ende der Schwungphase eine Anhebung der Flexionsdämpfung erfolgen, um ein übermäßiges Einbeugen des Unterteils 3 zu vermeiden. Durch die Veränderung der Flexionsdämpfungseinstellung in der Dämpfereinheit 50 ist es möglich, in Verbindung mit der Motorsteuerung eine effektive und sichere sowie einfache Steuerung der Energieeinbringung zur Bewegungsunterstützung durchzuführen.

Neben der dargestellten Ausführungsform als Flexionsunterstützung kann die Vorrichtung grundsätzlich auch zur Extensionsunterstützung eingesetzt werden, die Ausführungen zur Flexionsunterstützung gelten entsprechend auch für eine Extensionsunterstützung, wobei es auch möglich und vorgesehen ist, dass eine Flexions- und Extensionsunterstützung gemeinsam in einer Gelenkeinrichtung angeordnet sind.

## Patentansprüche

1. Verfahren zur Steuerung einer orthopädietechnischen Gelenkeinrichtung einer unteren Extremität, die Gelenkeinrichtung weist ein Oberteil (2) und ein gelenkig daran gelagertes Unterteil (3) auf, zwischen dem Oberteil (2) und dem Unterteil (3) ist eine Energieumwandlungseinrichtung (5) angeordnet, über die während des Gehens kinetische Energie aus der Relativbewegung zwischen dem Unterteil (3) und dem Oberteil (2) umgewandelt oder gespeichert und dem Gelenk wieder zugeführt wird, um die Relativbewegung zu unterstützen, wobei innerhalb eines Bewegungszyklus kinetische Energie umgewandelt und/oder gespeichert und innerhalb desselben Bewegungszyklus der Gelenkeinrichtung (1) gesteuert zeitversetzt als kinetische Energie wieder zugeführt wird, **dadurch gekennzeichnet, dass** die Zufuhr gespeicherter Energie dadurch verändert wird, dass einem Energiespeicher (54) extern Energie zugeführt oder entzogen wird.

2. Verfahren nach Anspruch 1, **dadurch gekennzeichnet, dass** kinetische Energie während einer Extensionsbewegung umgewandelt und/oder gespeichert wird und bei der Einleitung der Schwungphase zur Unterstützung der Flexion, nach der Zehenablösung zur Aufrechterhaltung der Flexionsgeschwindigkeit, nach Erreichen des maximalen Flexionswinkels zur Unterstützung der Extensionsbewegung und/oder nach der Flexionsbewegung nach dem initialen Fersenkontakt zur Unterstützung der Extensionsbewegung wieder zugeführt wird.

3. Verfahren nach Anspruch 1 oder 2, **dadurch gekennzeichnet, dass** kinetische Energie während der Flexionsbewegung umgewandelt und/oder gespeichert wird und zur Einleitung der Schwungphase zur Unterstützung der Flexionsbewegung und/oder zur Aufrechterhaltung der Beugegeschwindigkeit nach der Zehenablösung wieder zugeführt wird.

4. Verfahren nach Anspruch 3, **dadurch gekennzeichnet, dass** die kinetische Energie nach Einleitung der Schwungphase umgewandelt und/oder gespeichert wird und nach Erreichen der maximalen Flexionsgeschwindigkeit zur Unterstützung der Beugebewegung wieder zugeführt wird.

5. Verfahren nach einem der voranstehenden Ansprüche, **dadurch gekennzeichnet, dass** kinetische Energie vor Erreichen des Streckanschlages umgewandelt und/oder gespeichert und zur Einleitung und/oder Unterstützung der Flexion der Gelenkeinrichtung wieder zugeführt wird.

6. Verfahren nach einem der voranstehenden Ansprüche, **dadurch gekennzeichnet, dass** kinetische Energie während der Standphase zu Beginn der Standphasenflexion bei Fersenbelastung, vor dem Erreichen des Extensionsanschlages und/oder nach der Einleitung der Standphasenflexion bei Vorfußbelastung umgewandelt und/oder gespeichert wird.

7. Verfahren nach einem der voranstehenden Ansprüche, **dadurch gekennzeichnet, dass** die umgewandelte Energie in einem Bewegungszyklus vollständig der Gelenkeinrichtung wieder zugeführt wird.

8. Verfahren nach einem der voranstehenden Ansprüche, **dadurch gekennzeichnet, dass** die Speicherung und/oder Umwandlung der kinetischen Energie nur in vorbestimmten Phasen während eines Bewegungszyklus durchgeführt wird.

9. Verfahren nach Anspruch 1, **dadurch gekennzeichnet, dass** dem Energiespeicher (54) ein Aktuator (10) zugeordnet ist, über den der Energiespeicher (54) auf ein Mindestniveau befüllt wird, wenn die Relativbewegung dazu nicht ausreicht.

10. Verfahren nach einem der voranstehenden Ansprüche, **dadurch gekennzeichnet, dass** die Energie in Abhängigkeit von zumindest einem folgenden Kriterium oder einer Kombination mehrerer folgender Kriterien zugeführt oder entzogen wird:
- der Winkelstellung des Oberteils (2) zu dem Unterteil (3),
- der Position des Oberteils (2) und/oder des Unterteils (3) im Raum,
- einer Winkelgeschwindigkeit des Oberteils (2) und/oder des Unterteils (3),
- der Relativgeschwindigkeit zwischen Oberteil (2) und Unterteil (3),
- der Belastungssituation,
- der Beschleunigung des Oberteils (2) und/oder des Unterteils (3).

11. Verfahren nach einem der voranstehenden Ansprüche, **dadurch gekennzeichnet, dass** der Zeitpunkt eines in Eingriff Tretens der Umwandlungseinrichtung (5) zur Veränderung der umzuwandelnden Energiemenge und/oder der zugeführten Energiemenge verstellt wird.

12. Verfahren nach einem der voranstehenden Ansprüche, **dadurch gekennzeichnet, dass** der Energiespeicher (54) von einem Aktuator (10) aufgeladen wird, wenn die Umwandlungseinrichtung (5) nicht aufgrund der Relativbewegung zwischen dem Oberteil (2) und dem Unterteil (3) aktiv ist.

## Claims

1. A method for controlling an orthopedic joint device of a lower extremity; the joint device has an upper part (2) and a lower part (3) mounted in an articulated manner thereon; arranged between the upper part (2) and the lower part (3) is an energy conversion device (5), by way of which kinetic energy from the relative movement between the lower part (3) and the upper part (2) is converted or stored and fed back to the joint during the walking in order to assist the relative movement, wherein within a movement cycle, kinetic energy is converted and/or stored and, within the same movement cycle, is fed back to the joint device (1) in a controlled manner after a time delay as kinetic energy, **characterized in that** the supply of stored energy is changed by energy being externally supplied to or drawn from an energy store (54).

2. The method as claimed in claim 1, **characterized in that** kinetic energy is converted and/or stored during an extension movement and is fed back in the initiation of the swinging phase to assist the flexion, after the lifting off of the toe to maintain the flexion velocity, after reaching the maximum flexion angle to assist the extension movement and/or after the flexion movement following the initial heel contact to assist the extension movement.

3. The method as claimed in claim 1 or 2, **characterized in that** kinetic energy is converted and/or stored during the flexion movement and is fed back for initiating the swinging phase to assist the flexion movement and/or to maintain the bending velocity after the lifting off of the toe.

4. The method as claimed in claim 3, **characterized in that** the kinetic energy is converted and/or stored after initiation of the swinging phase and is fed back to assist the bending movement after reaching the maximum flexion velocity.

5. The method as claimed in one of the preceding claims, **characterized in that** kinetic energy is converted and/or stored before reaching the extension stop and is fed back for initiating and/or assisting the flexion of the joint device.

6. The method as claimed in one of the preceding claims, **characterized in that** kinetic energy is converted and/or stored during the standing phase at the beginning of the standing phase flexion with heel loading, before reaching the extension stop and/or after initiating the standing phase flexion with forefoot loading.

7. The method as claimed in one of the preceding claims, **characterized in that** the converted energy is completely fed back to the joint device in one movement cycle.

8. The method as claimed in one of the preceding claims, **characterized in that** the storage and/or conversion of the kinetic energy is only carried out in predetermined phases during a movement cycle.

9. The method as claimed in claim 1, **characterized in that** the energy store (54) is assigned an actuator (10), by way of which the energy store (54) is filled to a minimum level if the relative movement is not sufficient for this.

10. The method as claimed in one of the preceding claims, **characterized in that** the energy supplied or drawn in dependence on at least one following criterion or a combination of a number of the following criteria:
- the angular position of the upper part (2) in relation to the lower part (3),
- the position of the upper part (2) and/or the lower part (3) in space,
- an angular velocity of the upper part (2) and/or the lower part (3),
- the relative velocity between the upper part (2) and the lower part (3),
- the loading situation,
- the acceleration of the upper part (2) and/or the lower part (3).

11. The method as claimed in one of the preceding claims, **characterized in that** the point in time of an intervention of the conversion device (5) for changing the amount of energy to be converted and/or the amount of energy supplied is adjusted.

12. The method as claimed in one of the preceding claims, **characterized in that** the energy store (54) is charged by an actuator (10) if the conversion device (5) is not active on account of the relative movement between the upper part (2) and the lower part (3).

## Revendications

1. Procédé pour commander un dispositif formant articulation orthopédique d'une extrémité inférieure, ledit dispositif formant articulation comportant une partie supérieure (2) et une partie inférieure (3) qui est montée en articulation sur celle-ci, entre ladite partie supérieure (2) et ladite partie inférieure (3) est agencé un moyen de conversion d'énergie (5) par lequel, pendant la marche, de l'énergie cinétique issue du mouvement relatif entre la partie inférieure (3) et la partie supérieure (2) est convertie ou stockée et est retournée à l'articulation, afin de soutenir le mouvement relatif, et à l'intérieur d'un cycle de mouvement l'énergie cinétique est convertie et/ou stockée et est retournée de façon commandée et décalée dans le temps sous forme d'énergie cinétique au dispositif formant articulation (1) à l'intérieur du même cycle de mouvement,
**caractérisé en ce que** l'apport de l'énergie stockée est modifié du fait que de l'énergie est amenée par voie externe à un accumulateur d'énergie (54) ou bien en est retirée.

2. Procédé selon la revendication 1, **caractérisé en ce que** l'énergie cinétique est convertie et/ou stockée pendant un mouvement d'extension et est retournée lors de l'initialisation de la phase pendulaire pour soutenir la flexion après détachement des orteils en vue de maintenir la vitesse de flexion, une fois que l'angle de flexion maximal est atteint, en vue de soutenir le mouvement d'extension, et/ou après le mouvement de flexion à l'issue du contact initial du talon, en vue de soutenir le mouvement d'extension.

3. Procédé selon la revendication 1 ou 2, **caractérisé en ce que** l'énergie cinétique est convertie et/ou stockée pendant le mouvement de flexion et est retournée lors de l'initialisation de la phase pendulaire pour soutenir le mouvement de flexion et/ou pour maintenir la vitesse de flexion après détachement des orteils.

4. Procédé selon la revendication 3, **caractérisé en ce que** l'énergie cinétique est convertie et/ou stockée après l'initialisation de la phase pendulaire et est retournée une fois que la vitesse de flexion maximale est atteinte, en vue de soutenir le mouvement de flexion.

5. Procédé selon l'une des revendications précédentes, **caractérisé en ce que** l'énergie cinétique est convertie et/ou stockée avant d'atteindre la butée d'extension et est retournée pour initialiser et/ou soutenir la flexion du dispositif formant articulation.

6. Procédé selon l'une des revendications précédentes, **caractérisé en ce que** l'énergie cinétique est convertie et/ou stockée pendant la station debout au début de la flexion en station debout lorsque le talon est chargé, avant d'atteindre la butée d'extension et/ou après l'initialisation de la flexion en station debout lorsque l'avant-pied est chargé.

7. Procédé selon l'une des revendications précédentes, **caractérisé en ce que** l'énergie convertie est retournée complètement au dispositif formant articulation pendant un cycle de mouvement.

8. Procédé selon l'une des revendications précédentes, **caractérisé en ce que** le stockage et/ou la conversion de l'énergie cinétique est effectué(e) uniquement pendant des phases prédéterminées pendant un cycle de mouvement.

9. Procédé selon la revendication 1, **caractérisé en ce qu'**un actionneur (10) est associé à l'accumulateur d'énergie (54), par lequel l'accumulateur d'énergie (54) est rempli à un niveau minimum lorsque le mouvement relatif n'y suffit pas.

10. Procédé selon l'une des revendications précédentes, **caractérisé en ce que**
l'énergie est apportée ou retirée en fonction de l'un au moins des critères suivants ou d'une combinaison de plusieurs des critères suivants :
- la position angulaire de la partie supérieure (2) par rapport à la partie inférieure (3),
- la position de la partie supérieure (2) et/ou de la partie inférieure (3) dans l'espace,
- une vitesse angulaire de la partie supérieure (2) et/ou de la partie inférieure (3),
- la vitesse relative entre la partie supérieure (2) et la partie inférieure (3),
- la situation de charge,
- l'accélération de la partie supérieure (2) et/ou de la partie inférieure (3).

11. Procédé selon l'une des revendications précédentes, **caractérisé en ce que** l'instant d'une intervention du moyen de conversion (5) est réglé en vue de modifier la quantité d'énergie à convertir et/ou de la quantité d'énergie apportée.

12. Procédé selon l'une des revendications précédentes, **caractérisé en ce que** l'accumulateur d'énergie (54) est chargé par un actionneur (10) lorsque le moyen de conversion (5) n'est pas actif en raison du mouvement relatif entre la partie supérieure (2) et la partie inférieure (3).
